# NEW EUROPEAN PATENT SPECIFICATION

(11) **EP 0 935 969 B2**
(45) Date of publication and mention of the opposition decision: **08.09.2010**
(45) Mention of the grant of the patent: 17.08.2005
(21) Application number: 99102288.0
(22) Date of filing: 05.02.1999
(51) Int. Cl.: A61M 5/00, B65D 85/24, B65D 73/02

(54) **Flexible continuous strip package for medical syringes**
Verpackungen von einen endlosen weichen Streifen für medizinische Spritzen
Emballage d'une bande continue souple pour seringues medicales

(30) Priority: 10.02.1998 US 21425
(43) Date of publication of application: 18.08.1999
(73) Proprietor: Becton Dickinson and Company, Franklin Lakes, New Jersey 07417-1880 (US)
(72) Inventor: Timko, James J., Sparta, NJ 07871 (US); Prais, Alfred W., Hewitt, NJ 07421 (US); Morigi, Adriano, Rutherford, NJ 07070 (US)
(74) Representative: Molnia, David

(56) References cited:
- EP-A- 0 042 336
- EP-A- 0 180 341
- DE-A- 4 225 876
- US-A- 2 023 289
- US-A- 3 331 499
- US-A- 3 736 933
- US-A- 4 015 709
- US-A- 4 674 652
- US-A- 4 865 592
- US-A- 4 921 102
- US-A- 5 242 053

## Description

### BACKGROUND OF THE INVENTION

This invention generally relates to an arrangement for packaging and handling medical syringes. More specifically, this invention relates to an arrangement including a continuous flexible strip that maintains a plurality of syringes in a preselected alignment for packaging and handling purposes.

Hypodermic needle syringes are well known and come in a variety of configurations. One challenge associated with all hypodermic syringes is properly and efficiently packaging and handling the syringes during manufacturing and pharmaceutical filling operations. Although a variety of packaging and handling techniques and machinery have been developed or proposed, most of these tend to be specialized for handling only one specific type of syringe. Moreover, most existing systems tend to introduce undesirable expense because the machinery is overly complex or there tends to be an excessive amount of wasted material that is discarded or needs to be recycled. Further, current packaging and handling methods are often labor-intensive, which introduces additional expense in the manufacturing or packaging process.

One solution that has been developed by the assignee of this invention includes connecting a number of syringes together with integral web portions extending between the syringes. This solution is shown in United States Patent No. 4,865,592, which issued on September 12, 1989. While that solution is useful for maintaining a generally parallel alignment of syringes for packaging and pharmaceutical pre-filling operations, it is not without shortcomings and drawbacks.

In the arrangement shown in U.S. Patent No. 4,865,592, the web portions that extend between syringes are integrally formed with the syringes themselves and made of the same material. This introduces unnecessary expense since the plastic material used to make such a syringe is also used for the connecting web portions. As the web portions are later removed and discarded this introduces unnecessary waste and does not minimize expense. Further, the rigid web portions limit the number of syringes that can be included in a single package. Moreover, the arrangement of that patent is specialized in that it is only suitable for the specific type of syringe disclosed in that patent. Further, that arrangement requires the use of external tools or some device to separate the syringes when they are needed for

US-A-4,015,709 discloses a stiff package for holding an array of syringes. Because of the folded constitution of the walls of the package, the package is not flexible.

US-A-3,736,933 is directed to an applicator made of pliable material. The applicators are held together between a pair of sheets of paper or plastic. The sheets form pockets wherein the needle and the needle supporting portion are sitting.

US-A-5,242,053 discloses a carrier strip comprising voids for holding tubes. The plane of the strip is laying perpendicular to the longitudinal axis of the tube body When the strip is bended, the top or the bottom portions of neighboured tube bodies will collide.

There is a need for a syringe assembly arrangement that facilitates packaging and handling syringes during manufacturing and pharmaceutical filling processes. Further, there is a need for such an arrangement that is adaptable to be used with a variety of syringes, that is inexpensive and that avoids the introduction of undesirable waste material. It would be additionally advantageous to have an arrangement that is reusable and simple to work with under a variety of packaging or handling situations.

It is an object of the invention to improve the handling of a syringe assembly. This object is, according to the invention, achieved with the features of claim 1.

### SUMMARY OF THE INVENTION

In general terms, this invention is a syringe assembly that includes a flexible strip that interconnects a plurality of syringes and maintains them in a selected alignment for efficient and easy handling during packaging and syringe-filling processes. A syringe assembly designed according to this invention includes a plurality of syringes, which each have a barrel portion on one end and a needle supporting portion at a second end. A central axis extends along a center of the barrel and needle supporting portions. The syringes preferably are made from a substantially rigid plastic material. A supporting strip, which preferably is made from a substantially flexible material, engages a segment of each barrel portion such that the syringes are maintained in a preselected relative alignment where the central axis of each syringe is substantially parallel with those of the other syringes. Further, a preselected spacing is maintained between the syringes along a single syringe assembly.

The supporting strip of this invention includes a single strip of material having a plurality of barrel receiving portions where the syringe barrels are snap-fit into those portions and selectively maintained in the desired alignment by the supporting strip. The supporting strip includes two strips that are snap-fit together and capture the syringe barrel portions between the two strips so that the syringes are maintained in the desired alignment.

The various features and advantages of this invention will become apparent to those skilled in the art from the following detailed description of the preferred embodiment. The drawings that accompany the detailed description can be briefly described as follows.

### BRIEF DESCRIPTION OF THE DRAWINGS

Figure 1 is a diagrammatic illustration of a syringe assembly designed according to this invention.
Figure 2 diagrammatically illustrates selected details of the alternative of Figure 1.
Figure 3 is an embodiment of a supporting strip designed according to this invention.
Figure 4 is a diagrammatic illustration of an assembly process showing an alternative of a supporting strip designed according to this invention.
Figure 5 diagrammatically illustrates an arrangement of more than one syringe assembly designed according to this invention.
Figure 6 illustrates a method of packaging a plurality of syringe assemblies designed according to this invention.
Figure 7 illustrates another packaging arrangement that is useful with this invention.

### DETAILED DESCRIPTION OF THE PREFERRED EMBODIMENT

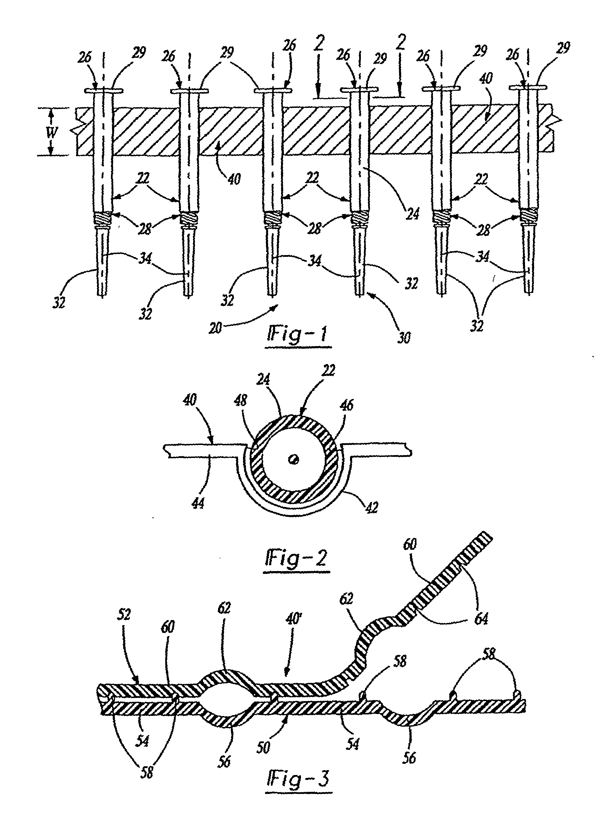

Figure 1 illustrates a syringe assembly 20 where a plurality of syringes 22 are maintained in a selected alignment relative to each other. Each syringe 22 includes a barrel portion 24 extending along a substantial portion of the syringe 22 from a first end 26. A flange 29 at the end of the barrel portion 24 facilitates utilizing the syringe 22 during a normal hypodermic injection procedure. A needle supporting portion 28 is located near a second end 30 of the syringe 22. In the illustrated embodiment, a needle cover 32 is in place over the needle of the hypodermic syringe. A central axis 34 extends through the center of the barrel portion 24 and the needle supporting portion 28.

The syringes 22 are maintained in the preselected alignment, which preferably includes the central axes 34 of the syringes 22 being substantially parallel, by a supporting strip 40. The supporting strip 40 preferably is made from a substantially flexible material. The supporting strip 40 preferably is arranged lengthwise perpendicular to the axes 34 of the syringes 22. The supporting strip 40 engages the barrel portions 24 of the syringes 22 in a manner to be described in more detail below. The supporting strip 40 has a width W that is great enough to selectively maintain the syringes 22 in the preferred relative alignment.

Figure 2 illustrates, in more detail, an alternative to the present invention of the supporting strip 40 from Figure 1. The supporting strip 40 includes a plurality of barrel receiving portions 42 and a plurality of connecting segments 44 extending between the barrel receiving portions 42. The barrel portion 24 of each syringe 22 preferably is snap-fit and received into a corresponding barrel receiving portion 42. An interior surface of the receiving portion 42 preferably includes ends 46 and 48 that engage the barrel portion 24 in a manner that maintains the syringe 22 in place on the supporting strip 40 until the syringe is selectively removed for use. The alternative of Figure 2 preferably includes a barrel receiving portion 42 that circumscribes more than one half of the barrel portion 24 so that the ends 46 and 48 are positioned to be effective to maintain the syringe 22 on the supporting strip 40.

Figure 3 illustrates an embodiment of the supporting strip 40, which is designated as 40' in Figure 3. In this embodiment, the supporting strip 40' includes a first strip 50 and a second strip 52. Each of the strips 50 and 52 preferably are made from the same, substantially flexible material. The preferred material of this embodiment is plastic.

The first strip 50 includes a plurality of connecting segments 54 extending between barrel receiving portions 56. The connecting segments 54 preferably include a plurality of snap projections 58 extending from an interior surface of the first strip 50. The second strip 52 preferably includes a corresponding plurality of interconnecting segments 60 that extend between barrel receiving portions 62. The interior surface of the connecting segments 60 preferably include detents 64 that engage corresponding projections 58 from the first strip 50. In this manner, the first strip 50 is snap-fit to the second strip 52 and the barrel portions 24 of the syringes 22 are received in the barrel receiving portions 56 and 62 as desired.

The embodiment of Figure 3 has the advantage of being useable with a large variety of syringes 22 and can be made from a number of suitable materials. The embodiment of Figure 3 also provides a somewhat more secure connection between the supporting strip 40' and the syringes 22 compared to the embodiment of Figures 1 and 2.

The embodiments of Figures 1 and 3 have the advantage of being reusable and allow for selective removal or replacement of any particular syringe 22 from the assembly 20.

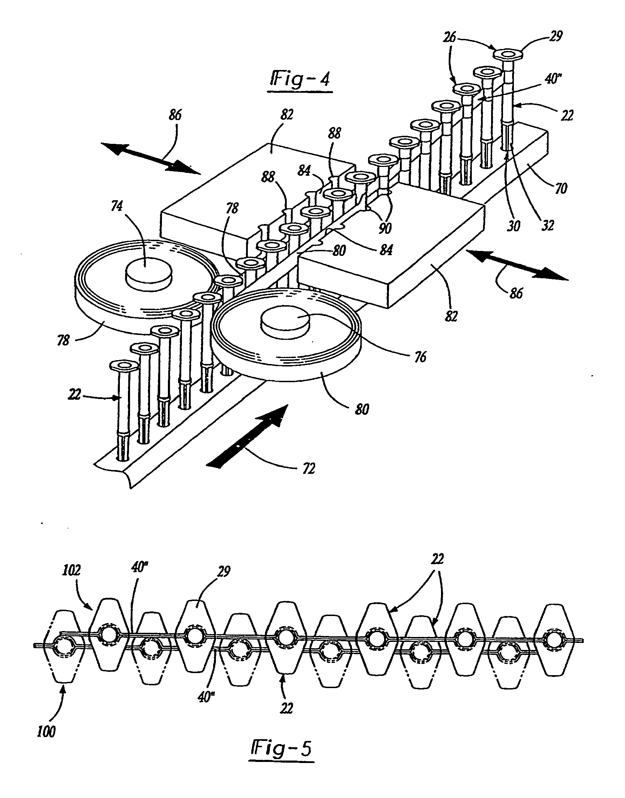

Figure 4 illustrates another alternative to the present invention of the supporting strip 40''. Figure 4 diagrammatically illustrates an assembly arrangement for making a syringe assembly 20. A plurality of syringes 22 are supported on a moving support member 70 that preferably moves under control of a computer (not illustrated) in the direction of arrow 72. The syringes 22 move relative to a pair of reels 74 and 76. The first reel 74 supports a roll 78 of material that is a first strip of the supporting strip 40''. Similarly, the reel 76 supports a roll 80, which is the second strip of the supporting strip 40''. The preferred material for the first strip 78 and the second strip 80 is plastic, however, a variety of materials such as a foil or other substantially flexible materials could be used.

The first strip 78 and the second strip 80 preferably are joined together at the segments that extend between the syringe barrels. An inside surface of at least one of the first strips 78 or the second strip 80 can include an adhesive useful for bonding the two strips together in the intermediate segment portions. Alternatively, an adhesive can be placed on the inside surface (i.e., that surface that faces toward the syringe barrels and toward the other strip during the assembly process). The adhesive can be heat-activated or a contact adhesive. Yet another alternative includes using a first strip 78 and a second strip 80 made from a plastic material that is easily melted together in a manner that the first strip 78 and the second strip 80 would be joined together without substantially deforming the plastic material.

A pair of moving members 82 each include an inner surface 84. The inner surfaces 84 actively engage the first strip 78 and the second strip 80 as the moving members 82 move inward and outward relative to the row of syringes 22 as illustrated by the arrows 86. The contour of the inner surfaces 84 preferably includes recesses 88 and 90 that nestingly receive the syringe barrels 22 as the moving members 82 move inward. Sufficient inward motion of the moving members 82 causes the first strip 78 to come into contact with the second strip 80 while simultaneously wrapping the first and second strips around the barrel portions of the syringes that are between the two moving members 82. In an embodiment where a contact adhesive is used to join the first strip 78 to the second strip 80, simply moving the moving members 82 toward each other so that the first and second strips come into contact is sufficient to secure the syringes 22 in the desired alignment supported by the supporting strip 40".

In an alternative where a heat-activated adhesive is used, the interior surfaces 84 on the moving members 82 can include appropriately heated surfaces or heating elements that will activate the adhesive and cause proper bonding so that the syringes 22 are supported in the selected alignment. Similarly, where heated material is fused or melted together to form the supporting strip 40'' the interior surfaces 84 include appropriate heated surfaces or heating elements to accomplish the desired joining of the first strip 78 and the second strip 80.

As can be appreciated from the illustration of Figure 4, four of the syringes 22 are secured between the first 78 and the second strip 80 each time that the moving members 82 move sufficiently inward to cause the first strip to be joined to the second strip. Further, as is shown in Figures 1 and 4, the supporting strip preferably is positioned near the first end 26 of the syringe 22. Placing the supporting strip about the barrel portion 24 is especially advantageous because it provides a more secure connection. The barrel portions 24 typically have a larger dimension than the needle supporting portions 22 and therefore provide more stable surfaces for connecting the syringes 22 to the supporting strip. Further, providing the connection about the barrel portion reduces the risk of damaging the needle supporting portion 28 of the syringes 22.

Under most circumstances, the syringes 22 preferably are removed from the supporting strip without the use of any external tools. In embodiments where the supporting strip includes two adhesively bonded strips (i.e., first strip 78 and second strip 80), when someone needs to remove a syringe 22 from the assembly 20, they simply separate the first and second strips. This operation would be similar to separating the first strip 50 and the second strip 52 of the embodiment of Figure 3. The difference between the alternative and the embodiment is that the adhesively bonded first and second strip, in most cases, cannot be manually rebonded together.

For the alternative examples where the first strip 78 and the second strip 80 are effectively fused or melted together, a plurality of scores or perforations 96 preferably are provided in the intermediate sections extending between the barrels of the syringes. The scores or perforations 96 facilitate easily removing a selected number of syringes from the assembly without the use of external tools. The scores 96 preferably are formed during the assembly process by providing the inner surfaces 84 of the moving members 82 with appropriate projections that will form the scores or perforations 96 in the supporting strip 40'' during the assembly process. Alternatively, the first strip 78 and the second strip 80 can be prescored or preperforated on the reels 74 and 76, respectively.

In the alternative examples where the supporting strip 40'' is secured about the barrel portions 24 in a manner where the barrel-receiving portions of the supporting strip remain on the syringes even after the syringes are removed from the assembly, the supporting strip material preferably is transparent so that any indicators marked on the syringe barrel portions are visible through the supporting strip.

Once a syringe assembly 20 is completed it provides the advantage of being readily usable in a prefilling operation. For example, high-speed pharmaceutical linear filling systems can readily receive an assembly 20 of a plurality of syringes 22. Further, the preselected relative alignment of the syringes 22 leaves them well-suited for oriented presentation into the prefilling system. Further, the syringes 22 can be sterilized in large quantities after they have been assembled into the syringe assemblies 20.

Another advantage to the assembly 20 is its ready usefulness for packaging a large number of syringes 22 for shipment or handling. Figure 5 illustrates how a first syringe assembly 100 can be nested together with a second syringe assembly 102). The syringes of the assembly 100 are interdigitated with the syringes of the assembly 102. In other words, the syringes of one assembly are placed in the intermediate spaces between the syringes of the other assembly.

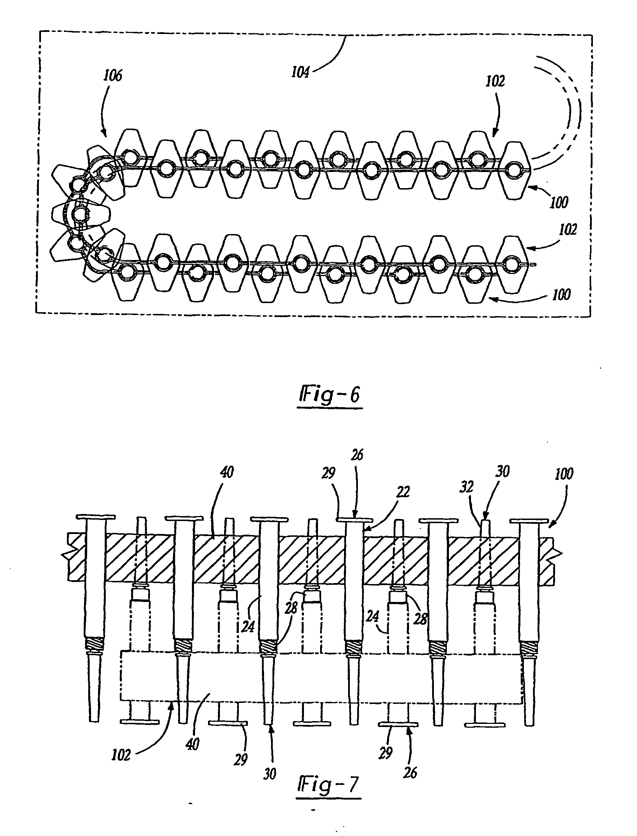

Figure 6 illustrates a preferred orientation of the two assemblies 100 and 102 within a package 104. The continuous, flexible supporting strip allows a large number of syringes to be placed within the package 104 in a folded pattern. The folded pattern 106 of Figure 6 is preferred generally S-shaped. This allows for easy packaging that minimizes the amount of bulk packaging material that needs to be used in handling a large number of syringes 22. The stability of the alignment of the syringes provided by the supporting strip 40 protects the syringes under most circumstances and reduces the amount of packaging material required. Further, the continuous supporting strip makes it easy for a selected amount of syringes 22 to be removed from a package without disturbing the entire arrangement of syringes on a repeated basis.

The most preferred alignment of a first assembly 100 and a second assembly 102 of syringes 22 is illustrated in Figure 7. In this orientation, the needle supporting portions 28 of one assembly are positioned facing in the same direction as the barrel portions 24 of the other syringe assembly. In other words, the first end 26 of each syringe 22 in the assembly 100 is adjacent the second end 30 of each syringe 22 in the second assembly 102. Such an arrangement maximizes packaging density and allows a greater number of syringes to be placed within any given package.

The use of a variety of generally flexible materials for the supporting strip of this invention makes it useful and applicable for a large variety of syringe types. Further, the inventive arrangement allows for easy access to a selected number of syringes without requiring the use of external tools or devices to remove the syringes from the assembly. Further, relatively inexpensive materials can be used to form the supporting strip and the amount of wasted material is minimized when a syringe assembly is designed according to this invention.

## Claims

1. A syringe assembly (20), comprising:
a plurality of syringes (22) made from a first, substantially rigid material, each of said plurality of syringes (22) having:
a barrel portion (24) having a first outside dimension at a first end (26) of said syringe (22);
a needle supporting portion (28) having a second outside dimension at a second end (30) of said syringe, said second outside dimension being smaller than said first outside dimension;
a central axis (34) extending along a center of said barrel and needle supporting portions (24,28); and
a support engaging said syringes (22) such that said syringes (22) are maintained in a preselected relative alignment and spacing between each adjacent syringe (22), said preselected alignment including a substantially parallel alignment of each said central axis (34) with a remainder of said axes (34),
**characterized by**
the support being a flexible supporting strip (40) made from a second, substantially flexible material, and
said flexible supporting strip (40) engaging at least a segment of each said barrel portion (24), wherein said flexible supporting strip (40') comprises two strips (50,52) of said substantially flexible material that are snap-fit together and said syringe barrel portions (24) are maintained between said two strips (50,52) when said strips (50,52) are snapped together.

2. The assembly of claim 1, wherein said flexible supporting strip (40) releasably maintains said syringes (22) in said preselected alignment or said flexible supporting strip (40) engages each said barrel portion (24) adjacent said first end (26) of each of said syringes (22).

3. The assembly of claim 1, wherein a first one (50) of said two strips (50,52) includes a plurality of projections (58) spaced along one side of said first strip (50) and a second one (52) of said two strips (50,52) includes a corresponding plurality of detent members (64) that received respective ones of said projections (58) to maintain said first strip (50) adjacent said second strip (52) and to selectively maintain selected ones of said plurality of said syringes (22) in said preselected relative alignment.

4. The assembly of claim 2, wherein said flexible supporting strip (40) is reusable such that a selected number of syringes (22) can be removed from respective barrel receiving portions (42) and said barrel receiving portions (42) subsequently selectively receive and maintain said syringes (22) in said relative alignment.

## Patentansprüche

1. Spritzenvorrichtung (20) mit:
mehreren Spritzen (22) aus einem ersten im wesentlichen starren Material, wobei jede der mehreren Spritzen (22) aufweist:
einen Zylinderteil (24) mit einer ersten Außenabmessung an einem ersten Ende (26) der Spritze (22);
einen Nadelhalteteil (28) mit einer zweiten Außenabmessung an einem zweiten Ende (30) der Spritze, wobei die zweite Außenabmessung kleiner ist als die erste Außenabmessung;
eine Mittelachse (34), die durch die Mitte des Zylinder- und des Nadelhalteteils (24,28) verläuft; und
einen Halter, der derart an den Spritzen (22) angreift, dass die Spritzen (22) in einer vorgewählten relativen Ausrichtung und Beabstandung zu jeder benachbarten Spritze (22) gehalten werden, wobei die vorgewählte Ausrichtung eine im wesentlichen parallele Ausrichtung jeder Mittelachse (34) zu den übrigen Achsen (34) umfasst,
**dadurch gekennzeichnet, dass**
der Halter ein flexibler Haltestreifen (40) aus einem zweiten im wesentlichen flexiblen Material ist, und
der flexible Haltestreifen (40) zumindest an einem Segment jedes Zylinderteils (24) angreift, wobei der flexible Haltestreifen (40) zwei Streifen (50,52) aus dem im wesentlichen flexiblen Material aufweist, die zusammengreifen, und die Spritzenzylinderteile (24) zwischen den beiden Streifen (50,52) gehalten werden, wenn die Streifen (50,52) zusammengreifen.

2. Vorrichtung nach Anspruch 1, bei der der flexible Haltestreifen (40) die Spritzen (22) lösbar in der vorgewählten Ausrichtung hält oder der flexible Haltestreifen (40) benachbart zu dem ersten Ende (26) jeder Spritze (22) an jedem Zylinderteil (24) angreift.

3. Vorrichtung nach Anspruch 1, bei der ein erster (50) der beiden Streifen (50,52) mehrere Vorsprünge (58) aufweist, die voneinander beabstandet auf einer Seite des ersten Streifens (50) angeordnet sind, und bei der ein zweiter (52) der beiden Streifen (50,52) mehrere entsprechende Einrastelemente (64) aufweist, die jeweils einen der Vorsprünge (58) aufnehmen, um den ersten Streifen (50) benachbart zu dem zweiten Streifen (52) zu halten und ausgewählte Spritzen der mehreren Spritzen (22) in der vorgewählten relativen Ausrichtung zu halten.

4. Vorrichtung nach Anspruch 2, bei der der flexible Haltestreifen (40) derart wiederverwendbar ist, dass eine ausgewählte Anzahl von Spritzen (22) aus jeweiligen Zylinder-Aufnahmeteilen (42) entnommen werden kann und die Zylinder-Aufnahmeteile (42) anschließend die Spritzen (22) selektiv aufnehmen und in der relativen Ausrichtung halten.

## Revendications

1. Ensemble de seringues (20) comprenant:
une pluralité de seringues (22) réalisées à partir d'un premier matériau sensiblement rigide, chaque seringue de ladite pluralité de seringues (22) comprenant:
une partie de corps cylindrique (24) ayant une première dimension extérieure au niveau d'une première extrémité (26) de ladite seringue (22);
une partie de support d'aiguille (28) ayant une seconde dimension extérieure au niveau d'une seconde extrémité (30) de ladite seringue, ladite seconde dimension extérieure étant inférieure à ladite première dimension extérieure ;
un axe central (34) s'étendant le long d'un centre dudit corps 15 cylindrique et des parties de support d'aiguille (24, 28) ; et
un support mettant en prise lesdites seringues (22) de sorte que lesdites seringues (22) sont maintenues dans un alignement et un espacement relatifs présélectionnés entre chaque seringue (22) adjacente, ledit alignement présélectionné comprenant un alignement sensiblement parallèle de chaque axe central (34) avec un reste desdits axes (34),
**caractérisé par le fait que**
le support est une bande de support flexible (40) réalisée à partir d'un second matériau sensiblement flexible, et
ladite bande de support flexible (40) met en prise au moins un segment de chaque partie de corps cylindrique (24), dans lequel ladite bande de support flexible (40) comprend deux bandes (50, 52) dudit matériau sensiblement flexible qui sont enclenchées ensemble, et lesdites parties de corps cylindrique de seringue (24) sont maintenues entre lesdites deux bandes (50, 52) lorsque lesdites bandes (50, 52) sont enclenchées ensemble.

2. Ensemble selon la revendication 1, dans lequel ladite bande de support flexible (40) maintient de manière amovible lesdites seringues (22) dans ledit alignement présélectionné ou bien ladite bande de support flexible (40) met en prise chaque partie de corps cylindrique (24) adjacente à ladite première extrémité (26) de chacune desdites seringues (22).

3. Ensemble selon la revendication 1, dans lequel une première bande (50) desdites deux bandes (50, 52) comprend une pluralité de saillies (58) espacées le long d'un côté de ladite première bande (50) et une seconde bande (52) desdites deux bandes (50, 52) comprend une pluralité correspondante d'éléments de détente (64) qui reçoivent les saillies respectives desdites saillies (58) pour maintenir ladite première bande (50) 20 adjacente à ladite seconde bande (52) et pour maintenir de manière sélective les seringues de ladite pluralité desdites seringues (22) dans ledit alignement relatif présélectionné.

4. Ensemble selon la revendication 2, dans lequel ladite bande de support flexible (40) est réutilisable de sorte qu'un nombre sélectionné de seringues (22) peut être retiré des parties de réception de corps cylindrique (42) respectives et lesdites parties de réception de corps cylindrique (42) reçoivent et maintiennent par la suite de manière sensiblement sélective lesdites seringues (22) dans ledit alignement relatif.
